# EUROPÄISCHE PATENTSCHRIFT

(11) **EP 2 179 053 B1**
(45) Veröffentlichungstag und Bekanntmachung des Hinweises auf die Patenterteilung: **07.06.2017**
(21) Anmeldenummer: 08784292.8
(22) Anmeldetag: 07.07.2008
(51) Int. Cl.: C12Q 1/37

(54) **BESTIMMUNG DER AKTIVITÄT VON PROTEASEN**
DETERMINATION OF THE ACTIVITY OF PROTEASES
DÉTERMINATION DE L'ACTIVITÉ DE PROTÉASES

(30) Priorität: 06.07.2007 DE 102007031706; 20.07.2007 DE 102007033850
(43) Veröffentlichungstag der Anmeldung: 28.04.2010
(73) Patentinhaber: Papst Licensing GmbH & Co. KG, 78112 St. Georgen (DE)
(72) Erfinder: HOFER, Hans Werner, 78464 Konstanz (DE); MEIER, Hans Jörg, 78467 Konstanz (DE)
(74) Vertreter: WSL Patentanwälte Partnerschaft mbB
(86) Internationale Anmeldenummer: PCT/DE2008/001104
(87) Internationale Veröffentlichungsnummer: WO 2009/006877

(56) Entgegenhaltungen:
- WO-A-97/00969
- WO-A-98/37226
- US-A1- 2003 186 345
- BISSELL E R ET AL: "SYNTHESIS AND CHEMISTRY OF 7-AMINO-4-(TRIFLUOROMETHYL)COUMARIN AND ITS AMINO ACID AND PEPTIDE DERIVATIVES", THE JOURNAL OF ORGANIC CHEMISTRY, AMERICAN CHEMICAL SOCIETY, US, vol. 45, no. 12, 1 January 1980 (1980-01-01), pages 2283-2287, XP002161939, ISSN: 0022-3263, DOI: 10.1021/JO01300A003

## Beschreibung

### Gegenstand der Erfindung

Die Erfindung betrifft ein Verfahren zur Bestimmung der Aktivität wenigstens einer Cystein-Protease in einer flüssigen unter Blutplasma und Blutserum ausgewählten Messprobe, welche neben der wenigstens einen Protease, deren Aktivität zu bestimmen ist, gegebenenfalls wenigstens einen zu der Protease korrespondierenden Proteaseinhibitor enthält.

### Hintergrund der Erfindung

Zur Bestimmung der Konzentration von Enzymen in Gewebehomogenaten oder in Körperflüssigkeiten, zum Beispiel im Blutserum, stehen enzymatische Tests und immunologische Tests (zum Beispiel ELISA) zur Verfügung.

In der vorhandenen medizinischen Literatur wird berichtet, dass beim Tumorwachstum, bei der Tumorinvasion und bei der Bildung von Metastasen lysosomale Proteasen beteiligt sind, wie z. B. die Cysteinproteasen Cathepsin B, H, L. Dabei konnte gezeigt werden, dass z. B. Cathepsin B von Tumorzellen in die Plasmamembran und in den extrazellulären Raum freigesetzt wird, wo diese Proteasen am Abbau und der Auflösung der extrazellulären Matrix mitwirken.

Da erhöhte Proteaseaktivitäten in von Tumoren befallenen Geweben und in Körperflüssigkeiten (Blut, Urin, Sputum, Cerebrospinalflüssigkeit etc.) bei Tumorpatienten auftreten, die unter den verschiedensten Arten von Krebs leiden, könnten solche Proteasen als Tumormarker sehr effektiv sein, um einerseits Diagnose und Prognose solcher Krankheiten zu erleichtern und andererseits als mögliche Ansatzpunkte für ein therapeutisches Eingreifen zu dienen. So wird z. B. Cathepsin B (Cysteinprotease) in der Literatur als Tumormarker diskutiert (Cancer Research 2005, 65 (19), Oct. 1/2005, S. 8608 ff. Kopitz et al.)

Eine zuverlässige Messung des Gehalts solcher Enzyme im Gewebe und in Körperflüssigkeiten ist sehr wünschenswert.

Prinzipiell stehen zur Bestimmung der Konzentration von Enzymen in biologischen Proben immunologische Verfahren (z. B. ELISA) und enzymatische Aktivitätsmessungen zur Verfügung.

Aus Clinical Cancer Research Vol. 3,1815-1822, Oct. 1997 (Kos et al) ist z. B. bekannt, Cathepsin B mit Hilfe des Elisa-Verfahrens (immunologisch) zu bestimmen.

Die hier betrachteten Enzyme, die Proteasen, zeichnen sich durch zwei Eigenschaften aus, die bei ihrer Konzentrationsbestimmung zu berücksichtigen sind:
1. Proteasen kommen in biologischen Proben gewöhnlich immer zusammen mit ihren Vorstufen, den Proenzymen, vor.
2. Proteasen sind in biologischen Proben durch körpereigene Inhibitoren ganz oder teilweise inhibiert.
Wie eigene Versuche mit Blutserum zeigen, ist dort vor der eigentlichen Aktivitätsmessung eine Deinhibierung vorzunehmen, wenn man z. B. die Aktivität von Cathepsin B in der Probe ermitteln soll.

Die immunologische Gehaltsbestimmung solcher Proteasen misst in der Probe auf Grund der Methode an sich immer nur die Summe aus aktivem Enzym, inhibiertem Enzym, denaturiertem Enzym und Proenzym.

Mit dem erfindungsgemäßen Verfahren, das die enzymatische Aktivitätsmessung betrifft, kann man hingegen die Werte von aktivem und inhibiertem Enzym separat messen, sofern in der biologischen Probe beide Enzymformen nebeneinander vorliegen.

Aus der EP 0 776 374 sind ein Verfahren und eine Vorrichtung zur Bestimmung von Enzymaktivität in biologischen Proben wie Gewebehomogenaten bekannt, wobei beispielhaft einem Cathepsin körpereigene Inhibitoren, die zur Superfamilie der Cystatine gehören, affinitätschromatographisch entzogen werden, indem man eine biologische Probe über eine Affinitätschromatographiesäule mit kovalent an Sepharose gebundenem Papain laufen lässt. Papain, bei dem es sich ebenfalls um eine Cysteinprotease handelt, besitzt eine höhere Bindungsaffinität zu Cystatinen als die Cathepsine, weshalb das Papain dem Cathepsin den Inhibitor entzieht.

In immunologischen Tests, wie dem ELISA, werden die in einer Probe vorhandenen Enzyme spezifisch mittels Antikörpern nachgewiesen. Der immunologische Test ist zwar in der Regel sensitiver als der enzymatische Test, jedoch unterscheiden die Antikörper, z. B. bei den Cystein-Proteasen, nicht zwischen aktivem Enzym, durch Inhibitoren gehemmtem Enzym, Proenzym und denaturiertem Enzym. Da es bei der Bestimmung von Enzymen in biologischen Proben in der Regel auf die von den Enzymen bereitgestellte Aktivität ankommt, weil diese letztendlich biologische Vorgänge auslöst oder katalysiert, ist der Aussagegehalt vieler immunologischer Tests, welche die vorgenannten Bestimmungsergebnisse liefern, für die medizinische Analytik und Diagnostik mangelhaft.

Aus der Literatur sind Cathepsin-Aktivitätsmessungen mit einem fluorogenen Substrat (AMC) bekannt, und zwar aus Gewebeproben (Homogenaten).

Jedoch liegen auch Messungen über Cathepsin-Aktivität mit AMC in Körperflüssigkeit (Cerebrospinale Flüssigkeit) vor.

In beiden Fällen wird über Messungen ohne Inhibitorentzug mit Z-Arg-Arg-AMC als flourogenem Substrat berichtet.

Die Angaben in der o. g. Literatur belegen, dass die Autoren meinen, in den Gewebeproben und Körperflüssigkeiten das gesamte Cathepsin gemessen zu haben.

Im Falle von Gewebeproben aber lässt sich die Gesamtmenge aus aktivem und inhibiertem Enzym enzymatisch nur nach vorheriger Deinhibierung messen.

Weitere Literaturstellen berichten von Messungen der Enzymaktivität im Blutserum.

Skrzydlewska, E. et al., " Evaluation of Serum Cathepsine B and D in Relation to clinicopathological staging of colorectar cancer ", World J. Gastroenterol. 2005, 11 (27), Seiten 4225 - 4229, beschreiben die enzymatische Bestimmung von Cathepsin B im Blutserum, wobei die enzymatische Abspaltung von p-Nitroanilin (pNA) aus Bz-DL-Arginin-pNA als Maß für die Enzymaktivität mit Hilfe einer optischen Messmethode bestimmt wird.

Siewinski, M. et al., "A comparison of Cysteine peptidase activity and their inhibitors in the blood serum of pregnant women", Pakistan Journal of Medical Sciences, 2004, 20 (4), Seiten 381 - 384, beschreiben Arbeiten zur fluorimetrischen Bestimmung der Enzymaktivität von Cathepsin B. Dabei wird das Fluorogen AMC (7-Amino-4-methyl-cumarin) aus dem Substrat Z-Arg-Arg-AMC abgespaltet.

In beiden Fällen wurden jedoch keine Kontrollen mit spezifischen Inhibitoren durchgeführt, die zeigen würden, dass die gemessene Freisetzung des Fluorophors tatsächlich von einer Cysteinprotease bzw. von Cathepsin B herrührt. Es fand auch jeweils keine Entfernung von Inhibitoren vor der Messung statt.

In eigenen Kontrollversuchen mit dem Einsatz des für die Cysteinproteasen spezifischen Inhibitors E64 und des für Cathepsin B spezifischen Inhibitors CA-074 (beide Inhibitoren sind synthetisch hergestellt und käuflich) wurde festgestellt, dass man in Blutseren von Tumorpatienten und gesunden Probanden nur nach vorheriger Deinhibierung eine dem Cathepsin B zukommende Proteaseaktivität messen kann, dass also alles im Blutserum befindliche Cathepsin B durch Cysteinproteaseinhibitoren gehemmt ist.

Gewebeproben als Ausgangsmaterial zur Bestimmung der Proteaseaktivität haben jedoch den Nachteil, dass man sie erst bei einer Operation oder durch Biopsie erhält, was eine beschwerliche Methode der Probengewinnung für die Tumorfrüherkennung ist. Gewebeproben werden üblicherweise erst in einem Stadium entnommen, wenn eine Tumorerkrankung bereits diagnostiziert wurde oder zumindest Hinweise darauf eine Erkrankung vermuten lassen. Es wäre daher wünschenswert, ein Verfahren zur Bestimmung der Konzentration der Proteasen in einer leicht zugänglichen biologischen Probe, wie Blut, Urin oder Sputum zu erhalten. Es hat sich jedoch gezeigt, dass viele Proteasen, die als Marker (insbesondere Tumormarker) in Frage kommen, im Blut durch entsprechende Inhibitoren gehemmt vorliegen.

### Aufgabe

Die Aufgabe der Erfindung bestand somit darin, ein Verfahren bereitzustellen, mit dem sich auf einfache Weise (und bereits in einem frühen Stadium einer relevanten Krankheit) mit größerer Genauigkeit als beim Stand der Technik, jedenfalls mit ausreichender Genauigkeit, die Konzentration von Proteasen in biologischen Proben und Flüssigkeiten, insbesondere im Blutserum, zuverlässig bestimmen lässt, wobei die Bestimmung auch solche Proteasen umfassen soll, die durch Bindung körpereigener (endogener) Inhibitoren gehemmt sind. Dabei werden denaturierte Formen und Vorläuferstufen der Proteasen nicht erfasst.

### Lösung

Die Aufgabe der vorliegenden Erfindung wird durch ein Verfahren gemäß Anspruch 1 gelöst. Bevorzugte und vorteilhafte Ausführungsformen sind in den abhängigen Ansprüchen 2 bis 12 definiert. In einer Ausführungsform der Erfindung ist die wenigstens eine Cystein-Protease aus der Familie der Cathepsine ausgewählt, vorzugsweise unter Cathepsinen B, H, K, L und / oder S. Besonders bevorzugt ist die wenigstens eine Protease Cathepsin B.

Die Bestimmung von Cathepsin B ist aus medizinischer Sicht von besonderer Bedeutung, da eine erhöhte Konzentration an Cathepsin B im Blut als Tumormarker in der Literatur bereits diskutiert wird (s. oben!). Die Flüssige Messprobe ist Blutplasma oder Blutserum. Besonders bevorzugt ist die flüssige Messprobe Blutserum.

Erfindungsgemäß umfaßt das Substrat für die wenigstens eine Cystein-Protease eine Di- oder Oligopeptidsequenz, an deren C-Terminus direkt oder über einen Linker ein Fluorogen gebunden ist, welches von der Protease abgespalten wird. Das Fluorogen wird in dem Verfahren bei der proteolytischen Reaktion vorzugsweise von der Di- oder Oligopeptidsequenz abgespalten.

Ein Beispiel für eine Dipeptidsequenz, welche von der Cysteinprotease Cathepsin B spezifisch erkannt und gespalten wird, ist Arg-Arg. Als Substrat eignet sich somit zum Beispiel Z-Arg-Arg-AFC, wobei Z eine Schutzgruppe darstellt, die an den N-Terminus der Peptid-Sequenz gebunden ist.

Es ist erfindungsgemäß besonders bevorzugt, wenn das ungespaltene Substrat, welches die Di- oder Oligopeptidsequenz und das Fluorogen umfasst, ein Maximum der Fluoreszenz-Emissions-Wellenlänge besitzt, das sich von dem Maximum der Fluoreszenz-Emissions-Wellenlänge des durch die Protease bei der proteolytischen Reaktion abgespaltenen Fluorogens um wenigstens 20 nm, vorzugsweise wenigstens 40 nm oder wenigstens 60 nm oder wenigstens 80 nm, besonders bevorzugt wenigstens 100 nm unterscheidet. (Falls das Emissionsspektrum kein ausgeprägtes Maximum aufweist, kann ein (z. B. statistisch) repräsentativer Wert des Emissionsspektrums auch alternativ gelten).

Sind die Wellenlängen bzw. die Wellenlängenmaxima der Fluoreszenz-Emission des ungespaltenen Substrats und des abgespaltenen Fluorogens gleich oder liegen sie eng beieinander, so werden bei der Messung sowohl die Eigenfluoreszenz des ungespaltenen Substrats als auch die Fluoreszenz-Emission des abgespaltenen Fluorogens erfaßt. Solche Substrate und Fluorophore mit gleichen Fluoreszenz-Emissions-Wellenlängen sind aus dem Stand der Technik bekannt. Bei diesen Substraten ist eine Messung trotz der übereinstimmenden Fluoreszenz-Emissions-Wellenlängen möglich, wenn die Intensität der Fluoreszenz-Emission des Fluorogens gegenüber derjenigen des ungespaltenen Substrats bei gleicher oder ähnlicher Wellenlänge deutlich stärker ist. (Die Verstärkung des Signals wird dann im Vergleich zu einer enzymfreien Negativkontrolle als ein Maß für die Enzymaktivität ausgewertet). Ein Nachteil solcher Substrate besteht darin, dass ein signifikantes Ergebnis erst bei starker enzymatischer Aktivität und somit einer sehr deutlichen Erhöhung der Fluoreszenz-Emission gemessen werden kann, da das Signal bei geringer enzymatischer Aktivität häufig zu schwach ist und sich nicht ausreichend stark von der Grundfluoreszenz des ungespaltenen Substrats unterscheidet. Das Signal geht im Hintergrundrauschen unter oder hebt sich zumindest nicht aussagekräftig davon ab.

Eine Verschiebung der Detektionswellenlänge der Fluoreszenz-Emission zwischen dem Substrat und dem abgespaltenen Fluorogen hat den besonderen Vorteil, dass bei dieser Wellenlänge im wesentlichen nur das von dem Substrat abgespaltene Fluorophor und damit nur eine tatsächlich stattgefundene enzymatische Reaktion erfaßt wird. Je weiter die Emissionswellenlänge des abgespaltenen Fluorophors von der Fluoreszenz-Emissions-Wellenlänge des Substrates entfernt ist, desto empfindlicher kann die Bestimmung der Proteaseaktivität sein. Erst nach Zugabe des Substrats beginnt eine zeitlineare Zunahme der Konzentration des abgespaltenen Fluorogens.

Bei der Enzymaktivitätsmessung von Proteasen mit dem AMC-Substrat im Blutserum kommt nun noch der erschwerende Umstand hinzu, dass das Blutserum selbst bei der Detektionswellenlänge von 460 nm für das enzymatisch abgespaltene AMC-Fluorogen eine starke Eigenfluoreszenz aufweist.

Aus eigenen Versuchen zur Bestimmung der Aktivität von Cathepsin B im Blutserum in Mikrotiterplatten mit Hilfe eines Fluoreszenzreaders mit dem AMC-Substrat geht hervor, dass sich in der vorgegebenen Messzeit bei 460 nm kein Messsignal aus der bei dieser Wellenlänge vorhandenen Hintergrundfluoreszenz abhebt, die sich aus der Eigenfluoreszenz des Blutserums und der Eigenfluoreszenz des AMC-Substrats zusammensetzt, die wiederum teilweise durch das Blutserum gequencht ist.

Überraschenderweise jedoch ergibt sich, dass mit dem AFC-Substrat bei 508 nm ein mit dem Fortgang der enzymatischen Reaktion linear ansteigendes Messsignal vorhanden ist, das der Fluoreszenzemission des bei der enzymatischen Reaktion abgespaltenen AFC-Fluorogens zuzuordnen ist.

Bei der Wellenlänge von 508 nm für die Fluoreszenzemission des AFC-Fluorogens ist die Eigenfluoreszenz des Serums weitaus geringer als bei 460 nm, und die Eigenfluoreszenz des AFC-Substrats ist bei 508 nm Null.

So erweist sich unter diesen Messbedingungen das AFC-Substrat zur Aktivitätsmessung von Cathepsin B im Blutserum als mindestens 10 mal empfindlicher als das AMC-Substrat und macht die Aktivitätsmessung von Cathepsin in Blutserum jedenfalls im Fluoreszensreader erst möglich.

Ein deshalb erfindungsgemäß besonders bevorzugt eingesetztes Substrat ist Z-Arg-Arg-AFC, bei dem das Fluorogen 7-Amino-4-Trifluormethylcumarin (AFC) (handelsüblich erhältlich) ist.

Bissell, E.R. et al., "Synthesis and Chemistry of 7-Amino-4-(trifluormethyl)-coumarin and its Amino Acids and Peptide Derivatives", J. Org. Chem. 1980, 45, Seiten 2283 - 2287, beschreiben die Synthese des fluorogenen Substrates AFC (7-Amino-4-trifluormethylcumarin).

Beispielsweise eignet sich für die Bestimmung der Konzentration der Cysteinprotease Cathepsin B ein Substrat aus dem Dipeptid Arg-Arg, an dessen C-Terminus das Fluorophor 7-Amino-4-Trifluormethylcumarin kovalent gebunden ist. Das Dipeptid Arg-Arg ist in dem Substrat am N-Terminus mit einer Schutzgruppe Z versehen. Dieses Substrat wird hierin nachfolgend auch als Z-RR-AFC bezeichnet. Das Substrat Z-RR-AFC besitzt eine Fluoreszenz-Emissions-Wellenlänge von etwa 400 nm, wogegen die Fluoreszenz-Emissions-Wellenlänge des reinen Fluorophors AFC bei 505 nm liegt.

Des Weiteren ist die Verwendung des Fluorophors 7-Amino-4-Methycumarin (AMC) bekannt, welches für die Aktivitätsmessung von Cathepsin B ebenfalls in Verbindung mit dem Dipeptid Arg-Arg als Substrat eingesetzt werden kann (Z-RR-AMC). Dieses Substrat hat jedoch den oben beschriebenen Nachteil, dass die Fluoreszenz-Emissions-Wellenlängen sowohl des Substrats Z-RR-AMC als auch des freien Fluorogens AMC bei der gleichen Wellenlänge von etwa 460 nm liegen. Eine Diskriminierung zwischen ungespaltenem Substrat und gespaltenem Fluorophor ist in diesem Fall daher nur über die Signalintensität möglich, nicht jedoch über die Emissionswellenlänge. Die Empfindlichkeit der Messung ist für manche Fälle bei vorausgehender Deinhibierung aber doch ausreichend; vor allem, wenn Messproben von größerer Enzymkonzentration (z. B. Gewebeproben) oder von größerem Volumen vorliegen. Die Verwendung eines Fluorimeters (mit 90° Winkel zwischen anregender und emittierter Strahlung) kann bei Einsatz von AMC zusätzlich - wegen höherer Empfindlichkeit gegenüber der Detektion in Durchsicht (Fluoreszenzreader) - vorteilhaft sein.

Aus der EP 0776 374 sind ein Verfahren und eine Vorrichtung zur Deinhibierung der Protease einer flüssigen Messprobe bekannt. Dabei wird die flüssige Messprobe durch eine Durchflusssäule geschickt, in der sich ein Sepharose-Gel befindet, an das die Inhibitorbindesubstanz kovalent gebunden ist. Bei dieser kontinuierlichen Affinitätschromatographie geht der Inhibitor des Enzyminhibitorkomplexes in der Messprobe auf die Inhibitorbindesubstanz über und aus der Säule wird die Messprobe mit Inhibitor-freiem Enzym eluiert, das dann der Aktivitätsmessung zugeführt wird.

Da Agarose-Gel weniger Protein unspezifisch adsorbiert als Sepharose-Gel, bringt die Verwendung von Agarose-Gel bei dieser affinitätschromatographischen Inhibitorabtrennung einen wesentlichen Vorteil bezüglich der Nachweisempfindlichkeit des anschließend in seiner Aktivität zu messenden deinhibierten Enzyms.

Ein weiterer großer Vorteil bringt aber der Ersatz des Sepharose- bzw. Agarose-Gels durch ein festes Trägermaterial, auf welchem die Inhibitorbindesubstanz kovalent oder nicht-kovalent (adsorptiv) gebunden ist. Die Verwendung einer Folie oder einer Membran als Trägermaterial für die Inhibitorbindesubstanz hat den entscheidenden Vorteil, dass das feste Trägermaterial einfach zu handhaben und anzuwenden ist. Das feste Trägermaterial in der Form einer Folie oder einer Membran kann mit der flüssigen Messprobe einfach durch Eintauchen des Trägermaterials in die Probe in Kontakt gebracht werden, ohne dass aufwändige Apparaturen und Aufbauten, wie beispielsweise eine Durchflusssäule oder ähnliches, erforderlich sind.

Nach erfolgter Deinhibierung kann dann das feste Trägermaterial mit der Inhibitorbindesubstanz, auf die der Inhibitor übergegangen ist, aus der Messprobe herausgezogen werden.

Die Inhibitorbindesubstanz kann dabei kovalent oder adsorptiv an das feste Trägermaterial gebunden sein. Die Verwendung eines Trägermaterials, an dem die Inhibitorbindesubstanz kovalent daran gebunden ist, ist besonders vorteilhaft, da in diesem Fall beim Kontakt des festen Trägermaterials mit der flüssigen Messprobe die Gefahr ausgeschlossen ist, dass sich Inhibitorbindesubstanz von dem festen Trägermaterial löst, in die Messprobe gelangt und das Messergebnis verfälscht.

Das Trägermaterial kann jedes Material sein, welches geeignet ist, eine Inhibitorbindesubstanz, die üblicherweise ein Peptid oder ein Protein ist, ausreichend fest kovalent oder adsorptiv zu binden.

In einer bevorzugten Ausführungsform der Erfindung umfasst das Trägermaterial Nylon oder Nitro-zellulose oder ist daraus hergestellt. Entsprechende Nylon- oder Nitrozellulosefolien oder - membrane, an welche sich Substanzen, insbesondere Peptide oder Proteine kovalent oder nicht-kovalent binden lassen, sind handelsüblich erhältlich.

Die Inhibitorbindesubstanz ist für die zu bestimmende Protease und insbesondere für den oder die in der Messprobe vorhandenen Inhibitoren speziell auszuwählen und kann hier nicht abschließend festgelegt werden. Für die Familie der Cysteinproteasen gilt: Ihre Inhibitoren binden an die pflanzliche Protease Papain mit höherer Affinität und Bindungsstärke als Cathepsin B, was auch durch eigene Versuche bestätigt wurde.

Somit kann der Inhibitorentzug aus der Messprobe auf einfache Weise sowohl in einem klinischen Labor in Mikrotiterplatten, aber auch direkt in der Arztpraxis (im Blutserum) ohne großen Aufwand durchgeführt werden.

Der Inhibitorentzug aus der Messprobe kann somit auf einfache Weise sowohl in einem klinischen Labor, aber auch direkt in der Arztpraxis ohne großen Aufwand durchgeführt werden. Besonders bevorzugt ist es, wenn die Inhibitorbindesubstanz kovalent an das Trägermaterial gebunden ist. Beim Inkontaktbringen des festen Trägermaterials mit der flüssigen Messprobe ist dann die Gefahr ausgeschlossen, dass sich die Inhibitorbindesubstanz von dem festen Trägermaterial löst und in die Messprobe gelangt und darin das Messergebnis verfälschen kann. Das Trägermaterial kann jedes Material sein, welches geeignet ist, eine Inhibitorbindesubstanz, welche üblicherweise ein Oligopeptid oder ein Protein ist, ausreichend fest kovalent oder adsorptiv zu binden.

Die Inhibitorbindesubstanz ist für die zu bestimmende Protease und insbesondere den oder die in der Messprobe vorhandenen Inhibitor speziell auszuwählen und kann hier nicht abschließend festgelegt werden. Für die Familie der Cysteinproteasen gilt: Ihre Inhibitoren binden an die Pflanzenprotease Papain mit höherer Affinität und Bindungsstärke als Cathepsin B.

Die Erfindung wird nachfolgend anhand spezifischer Beispiele und Vergleichsversuche weiter beschrieben und näher erläutert. Die Begriffe Fluorophor und Fluorogen sollen gleiche Bedeutung haben.

### Beispiele:

Die Erfindung wird anhand von Beispielen und Untersuchungen mit der lysosomalen Cystein-Protease Cathepsin B im Blut bzw. im Blutserum weiter erläutert.

### Ergebnisse der Messungen:

### a. Messung der katalytischen Aktivität von Cathepsin B

### a 1. Einführung:

Die Aktivitätsmessung von Cathepsin B beruht auf der Spaltung eines Dipeptid-Substrats

Z-Arg-Arg-Fluorophor → Z-Arg-Arg + Fluorophor

(Z = Schutzgruppe, Arg = basische Aminosäure Arginin)

Für die empfindliche Messung der Aktivität von Cathepsin B wurden Fluoreszenz-Methoden mit zwei unterschiedlichen Fluorophoren eingesetzt, nämlich AMC (Biomol) und AFC (Biovision).

Das freie AMC fluoresziert bei der gleichen Wellenlänge wie das Substrat, aber mit höherer Fluoreszenzausbeute. Daher kommt es bei der katalytischen Spaltung zu einer Zunahme der Fluoreszenz.

Die auf AFC beruhende Methode hat eine erhöhte Sensitivität. Die erhöhte Sensitivität beruht auf der Verschiebung der Fluoreszenz des freien Fluorophors zu einer längeren Wellenlänge gegenüber der Wellenlänge des Substrats. Dadurch kann die Fluoreszenz des Substrats aus der Messung eliminiert und eine geringere "Hintergrundfluoreszenz" erreicht werden.

Als Sensitivität der Aktivitätsmessung wird allgemein ein Wert definiert, der ein mehrfaches der Differenz darstellt, also
Z mal (Messwert - Hintergrundswert)

Der Messwert muss vom Hintergrundwert "signifikant" unterschiedlich sein.

### a 2. Aktivitätsmessung:

Die Fluoreszenzmessungen wurden auf 96-well Mikrotiterplatten mit Hilfe von Fluoreszenzreadern (für Messungen mit AMC, Anregung bei 355nm, Fluoreszenz bei 450 nm) (für Messungen mit AFC, Anregung bei 400 nm, Fluoreszenz bei 508 nm) durchgeführt.

Bei der AMC-Methode erfolgten die Testreaktionen direkt bei Raumtemperatur auf der Microtiterplatte, die vorher durch Inkubation mit Albumin desensitiviert worden waren, um eine Bindung von Proteinen an den Kunststoff zu vermeiden.

Bei der AFC-Methode wurde der Ansatz zur Aktivitätsbestimmung in Reaktionsgefäßen bei 37°C durchgeführt. Der Inhalt des Reaktionsgefäßes wurde unmittelbar vor der Messung der Fluoreszenz in die Microtiterplatte überführt.

### a 3. Linearität der Fluoreszenz der freien Fluorogene AMC und AFC in Abhängigkeit von der Konzentration.

Ergebnis: Die Fluoreszenzen von AMC und AFC waren mit den verwendeten Testansätzen/Instrumenten linear (r² = 0.9999)

### a 4. Linearität der Aktivität von gereinigtem Cathepsin B in Abhängigkeit von der Enzymkonzentration mit beiden Substraten.

Ergebnis: Die Bestimmungen Aktivitäten von gereinigtem Cathepsin B waren linear abhängig von der eingesetzten Enzymkonzentration.

### a 5. Linearität der Aktivitätsbestimmung in Abhängigkeit von der Messzeit

Ergebnis: Die Freisetzung des Fluorophors vom Substrat war gemäß Messungen bis 90 min in zwei Testansätzen linear.

### b. Vorhandensein von Inhibitoren von Cathepsin B in humanem Serum.

Das Vorhandensein von Inhibitoren von Cathepsin B in humanen Seren ohne spezifische pathologische Befunde wurde mit Hilfe von gereinigtem Cathepsin B überprüft.

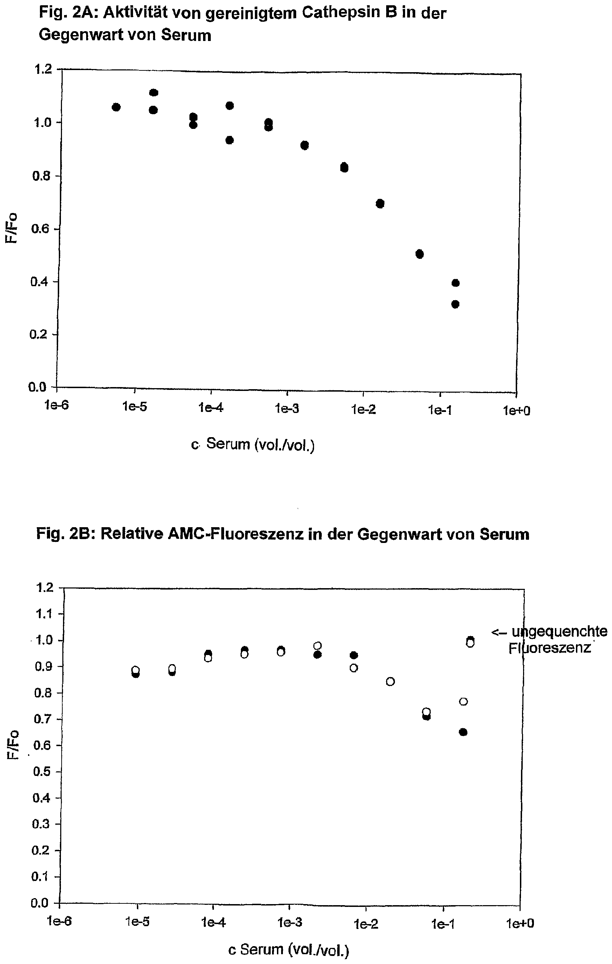

Ergebnis: Die scheinbare Aktivität von gereinigtem Cathepsin B nahm in Gegenwart von steigenden Konzentrationen von ("normalem") humanem Serum stark ab (bis auf <40% in Gegenwart von 10% Serum, s. Fig. 2a).

Eine Abnahme von 30-35% bei 10% Serum war auf eine "Fluoreszenz-Unterdrückung" (fluorescence quenching) durch die Eigenabsorption/Lichtzerstreuung des Serums bedingt, was durch den Einfluss von Serum auf die Fluoreszenz des freien AMC nachgewiesen wurde (s. Fig. 2b).

Für Untersuchungen in Abwesenheit von Serum war das AMC System jedoch nützlich.

### c. Hemmung von Cathepsin B durch Cystatin A.

Die Hemmung von Cathepsin B durch den bekannten Inhibitor Cystatin A wurde in vitro mit zwei unterschiedlichen Cathepsin B Konzentrationen untersucht.

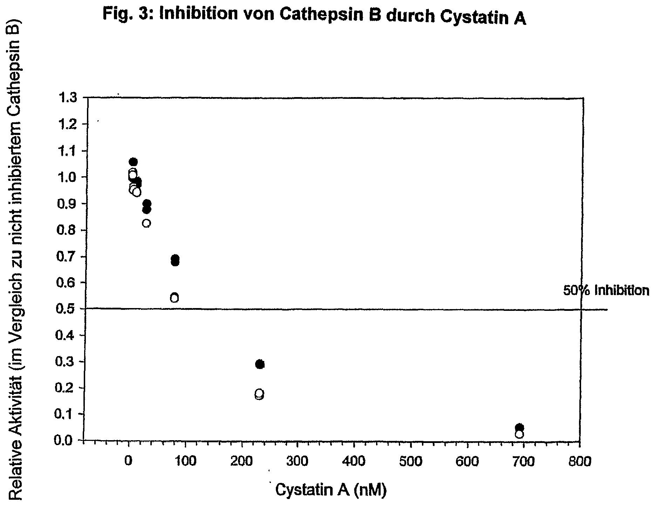

Ergebnis: Die Hemmung durch Cystatin A war nur wenig abhängig von der Cathepsin B Konzentration. (Fig. 3)

### d. Untersuchung einer Aufhebung der Hemmung von Cathepsin B durch Cystatin A mittels Behandlung mit immobilisiertem Papain.

Immobilisiertes Papain (kovalent gebunden an quervernetzte 6% Agarose-Sphären, beladen mit Papain.

Vorbehandlung: Ein Äquivalent der Suspension wurde mit 5-10 Äquivalenten Testpuffer 5 Mal gewaschen, wobei der Überstand durch Zentrifugation abgetrennt wurde. Nach dem letzten Waschen wurde das Sediment sorgfältig mit Fließpapier getrocknet und auf das ursprüngliche Suspensionsvolumen mit Testpuffer aufgefüllt.

Anmerkung: Es ist grundsätzlich zu unterscheiden zwischen der hier zunächst beschriebenen Papainbehandlung von reinem Cathepsin B und der später durchgeführten Behandlung von Serumproben, die auf Raumtemperatur und auf kurze Zeit angelegt war.

### d 1. Durchführung der Behandlung:

Die 50% Papain-Agarose Suspension wurde in entsprechenden Volumina in Polypropylenröhrchen gegeben, die feste Phase abzentrifugiert und der Überstand sorgfältig mit Pipette und Fließpapier abgenommen. Zur immobilisierten Phase wurden 160µl Enzymlösung gegeben und inkubiert.

Der Ansatz wurde zentrifugiert, ein Überstand wurde abgenommen und in den Test (entweder auf eine behandelte Mikrotiterplatte oder in 0.5 ml Röhrchen) überführt. Zu diesem Überstand wurde ein Volumen einer Puffer-Substralösung gegeben und 120 min inkubiert, gefolgt von der Messung im Fluorimeter.

### d 2. Zeitabhängigkeit der Stabilität von Cathepsin B in Gegenwart von Papain

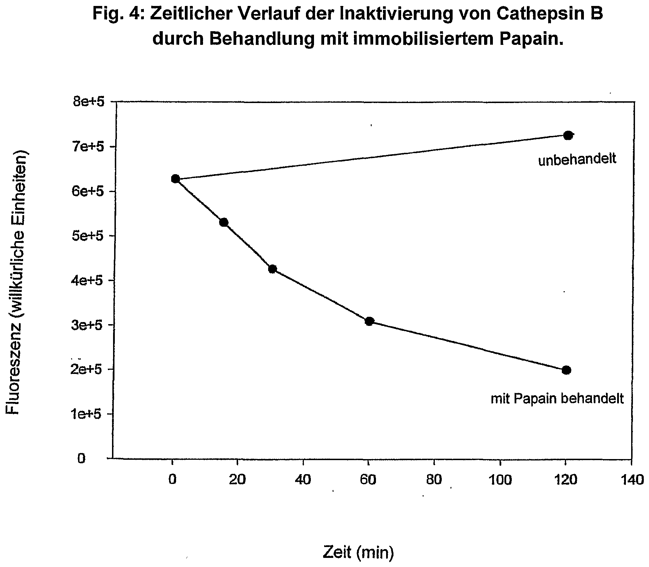

Die Stabilität von Cathepsin B in Gegenwart von immobilisiertem Papain wurde über einen Zeitraum von 120 min bei 4°C untersucht (Fig. 4).

Folgerung: Die Behandlung von Cathepsin B sollte mit einer möglichst kleinen Menge an immobilisiertem Papain über einen möglichst kurzen Zeitraum durchgeführt werden.

### d 3. Aufhebung der Hemmung von Cathepsin B durch Cystatin A durch Behandlung mit immobilisiertem Papain.

Die Aufhebung der Hemmung von Cathepsin B durch den definierten Inhibitor Cystatin A wurde bei steigenden Konzentrationen an immobilisiertem Papain in 2 unabhängigen Experimenten (5 und 10 ng Cathepsin B pro Ansatz, jeweils mit Doppelbestimmungen) bei 700 nM Cystatin A untersucht. Die Hemmung war bei dieser Konzentration an Inhibitor >95%.

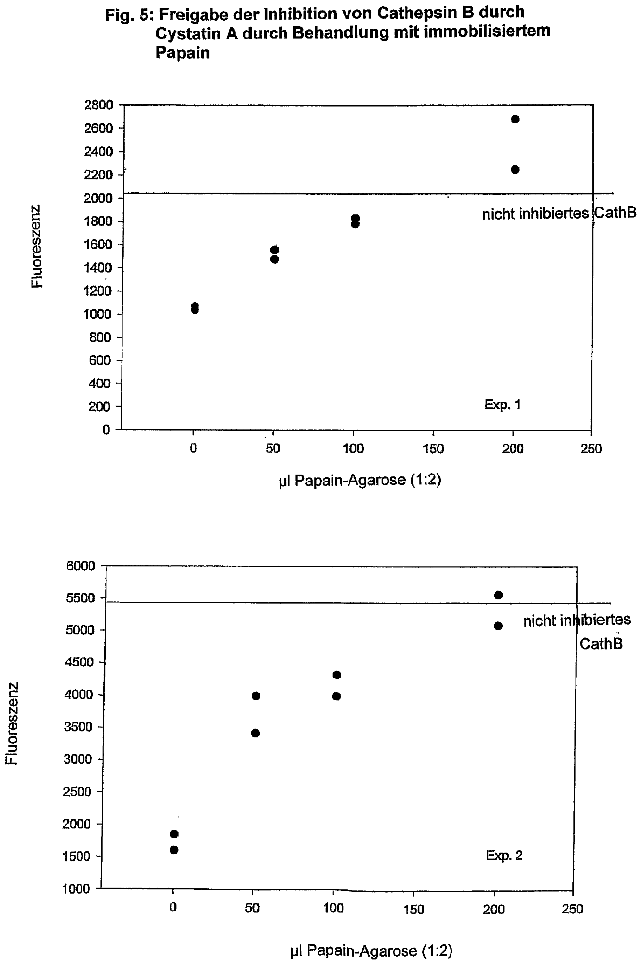

In beiden Experimenten wurde in Gegenwart von 100µl sedimentiertem Gel/Versuch (entsprechend 200µl an Suspension wie auf der Grafik aufgetragen) eine vollständige Aufhebung der Hemmung erreicht (Fig. 5).

Folgerung: Die Hemmung von Cathepsin B durch den definierten Inhibitor Cystatin A ist durch Behandlung mit geeigneten Mengen an immobilisiertem Papain aufhebbar.

### e. Hemmung von Cathepsin B durch Komponenten des Serums und Aufhebung der Hemmung durch Behandlung mit immobilisiertem Papain.

### e 1. Korrelation der Fluoreszenz mit der Cathepsin B-Konzentration in Abwesenheit bzw. Gegenwart von normalem Humanserum.

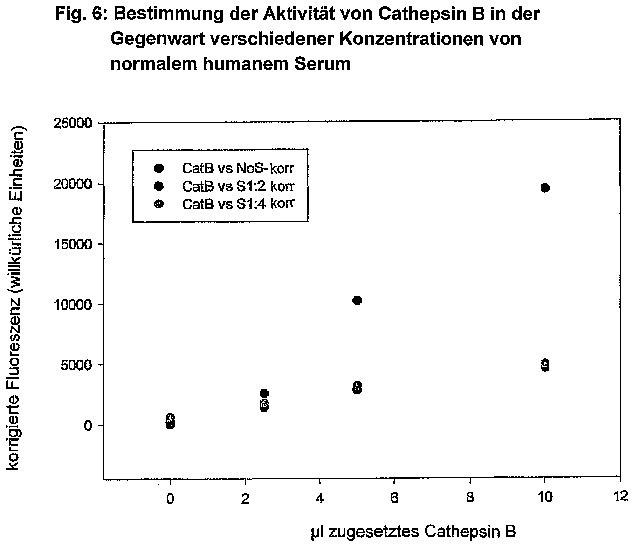

In Abwesenheit von Serum steigt das Testsignal von exogenem Cathepsin B ungefähr linear mit der zugesetzten Menge von Cathepsin B an (Fig. 6). In Gegenwart von Serum (Anteil entweder 50% oder 25% des Testvolumens) ist zwar ebenfalls eine lineare Abhängigkeit zu beobachten. Die Steigung beträgt jedoch nur ca. 25% derjenigen in Abwesenheit von Serum beobachteten.

Es tritt also bei niedrigen Cathepsin B Konzentrationen eine deutliche Hemmung der Aktivität durch Serum auf.

### e 2. Aufhebung der Hemmung der Cathepsin B Aktivität im Serum durch Behandlung mit immobilisiertem Papain

Die mit dem AFC Testsystem messbare "spontane" Proteaseaktivität im Serum war 0.12 +/- 0.02 nmol min⁻¹ ml⁻¹ ohne Vorbehandlung. Sie stieg nach 15 min. Inkubation mit immobilisiertem Papain um einen Faktor von 1.9 +/- 0.4 auf einen mittleren Wert von 0.23 +/- 0.03 an. Wurden den Ansätzen eine niedere Cathepsinaktivität von ca. 0.5 nmol min⁻¹ ml⁻¹ an (sie wurde also weitestgehend gehemmt). Nach Papainbehandlung war jedoch die nominelle Aktivität mit 0.52 nmol min⁻¹ ml⁻¹ voll messbar.

Das Serum enthält Bestandteile, welche sowohl endogene Cathepsin B ähnliche Proteasen als auch exogen zugesetztes Cathepsin B hemmen. Diese hemmenden Komponenten werden dem Serum durch Papainbehandlung entzogen.

### e 3. Wirkung des Cystein-Protease Hemmers E64 bzw. des Cathepsin B Inhibitors auf die spontane bzw. reaktivierte Protease Aktivität

Durch die Verwendung des unspezifischen Inhibitors E64 für Cysteinproteasen bzw. des Cathepsin B Inhibitors CA-074 sollte die Natur der als Cathepsin B Aktivität bestimmten Proteasen nachgewiesen werden.

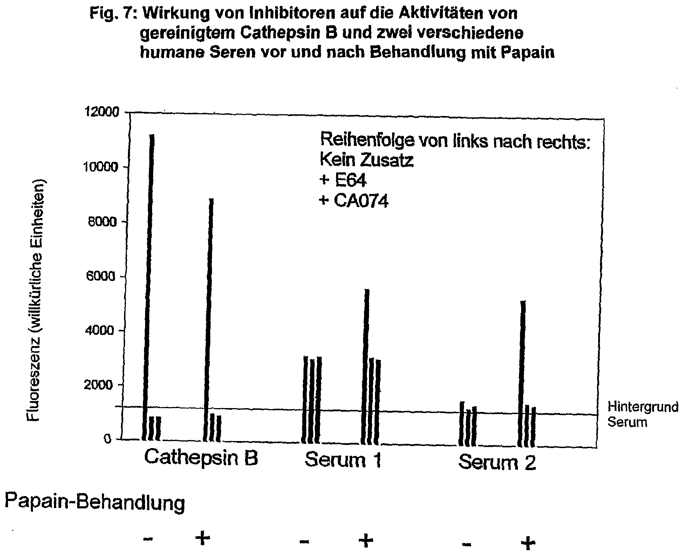

Untersucht wurden die enzymatischen Aktivitäten von Cathepsin B in geringer Konzentration und von zwei "normalen" humanen Seren mit und ohne Vorbehandlung mit immobilisiertem Papain (Fig. 7).

Die 15-minütige Vorbehandlung führte zu ca. 15% Verringerung der authentischen Cathepsin B Aktivität, wie dies auch in den anderen vergleichbaren Experimenten gesehen wurde. Durch beide Hemmstoffe wurde die Cathepsin B Aktivität (mit und ohne Vorbehandlung) vollständig gehemmt. (Der Hintergrund Wert der Fluoreszenz wurde in der Abbildung nicht von den Messwerten abgezogen, um eine objektive Einschätzung der Messempfindlichkeit zu erlauben.

In beiden Seren wurde ohne Vorbehandlung eine geringe proteolytische Aktivität gemessen. Diese Aktivitäten wurden weder durch E64 noch durch CA074 gehemmt. Sie sind also nicht auf Cathepsin B und aller Wahrscheinlichkeit nach auch nicht auf andere Cystein-Proteasen zurück zu führen.

Die Vorbehandlung mit immobilisiertem Papain führte bei beiden Seren zu einem Anstieg der Protease-Aktivität. Sowohl E64 als auch CA074 reduzierten diese Aktivität auf den Wert, der in den nicht vorbehandelten Proben gemessen wurde.

### Folgerung:

Ohne Vorbehandlung (d. h. Deinhibierung) mit immobilisiertem Papain war in beiden Seren keine Cathepsin B Aktivität nachweisbar.

### e 4. Zeitabhängigkeit der Einwirkung von immobilisiertem Papain auf die Cathepsin B Aktivität des Serums

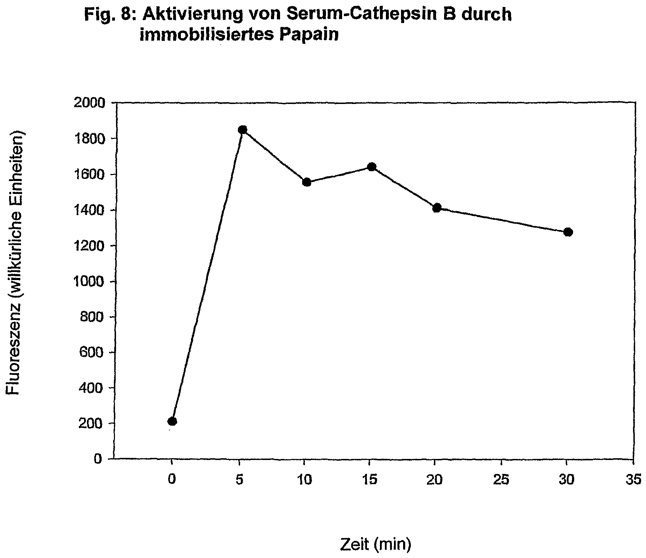

Mit dem Ziel, die Methodik der Vorbehandlung mit immobilisiertem Papain zu vereinfachen wurde die Zeitabhängigkeit der Aktivierung der Cathepsinaktivität im Serum bei Raumtemperatur untersucht. Der Verlauf der gemessenen Aktivitäten (die Hintergrundswerte wurden abgezogen) ist in Fig. 8 dargestellt. Der Wert beim Zeitpunkt 0 min entspricht der Messung ohne Vorbehandlung. Der Wert bei 5 min stellt den kürzesten Zeitwert dar, den die Prozeduren Inkubation (d.h. Zugabe der Probe zum immobiliisertem Papain), Zentrifugation und Probenentnahme im Routinebetrieb erfordern dürften. Zu diesem Zeitpunkt ist die gemessene Aktivität optimal.

Folgerung: Die Aktivierung von Cathepsin B im Serum ist ein sehr rascher Prozess.

### Figuren

Fig. 1
   Zeitlicher Verlauf der Fluoreszenzzunahme beim fluorimetrischen Aktivitätstest von Cathepsin B mit der AMC Methode. Cathepsin B (gereinigt aus der Milz vom Rind 10 units/ml) wurde in einer Verdünnung von -1/5000 in den Test eingesetzt. Die Kurven entsprechen zwei unabhängigen Testserien mit geringfügig unterschiedlichen Enzymaktivitäten.
Fig. 2
   Messung (a) der Cathepsin B Aktivität mit dem AMC Testsystem und (b) der AMC Fluoreszenz in Gegenwart von Serum.
   Die Messwerte wurden um die durch das AMC-Substrat und durch das Serum bedingten Hintergrund Fluoreszenzen korrigiert. Die Verdünnung des bovinen Cathepsins B in den Tests betrug 1/5000.
Fig. 3
   Hemmung der Aktivität von Cathepsin B durch steigende Konzentrationen an Cystatin A.
   Die Messungen wurden mit der AMC Methode durchgeführt. Die Cythepsin B Verdünnung in den Tests betrug 1/2500.
Fig. 4
   Zeitliche Abhängigkeit der Inaktivierung von Cathepsin B durch Behandlung mit immobilisiertem Papain.
   Die Messungen wurden mit der AMC Methode durchgeführt. Die Cythepsin B Verdünnung in den Tests betrug 1/2500.
Fig. 5
   Aufhebung der Hemmung von Cathepsin B durch Cystatin A durch steigende Mengen an immobilisiertem Papain.
   Die Tests wurden mit der AFC Methode durchgeführt.
Fig. 6
   Bestimmung der Aktivität von exogen zugesetztem Cathepsin B in Abwesenheit (schwarze Punkte) bzw. Anwesenheit von zwei unterschiedlichen Konzentrationen an humanem Serum.
   Die Tests wurden mit der AFC Methode durchgeführt.
Fig. 7
   Wirkung der Inhibitoren E64 und CA074 auf die Aktivität von gereinigtem Cathepsin B bzw. die Proteaseaktivitäten von Seren mit und ohne Behandlung mit immobilisiertem Papain.
   Die Messungen wurden mit der AFC Methode durchgeführt.
Fig. 8
   Zeitlicher Verlauf der Wirkung der Behandlung mit immobilisiertem Papain auf die Proteaseaktivität von humanem Serum.
   Die Messungen wurden mit der AFC Methode durchgeführt. Die Punkte sind die Mittelwerte von zwei Messserien.

Für die Durchführung der Aktivitätsmessung von Proteasen nach dem erfindungsgemäßen Verfahren im medizinischen Alltag (Praxis oder Klinikum) werden im Folgenden einige vorteilhafte Vorrichtungen beschrieben.

Aus dem Stand der Technik WO 97/00969 ist es bekannt, die Enzymaktivität solcher Enzyme, die in der Probe durch Inhibitoren vorwiegend inhibiert sind, dadurch zu messen, dass die Messprobe zuvor über eine Durchfluss- oder Durchlaufsäule gegeben wird, auf welcher der Probe die Inhibitoren entzogen werden, die das Enzym inhibieren. Danach wird das inhibitorfreie Enzym in eine Messzelle gegeben, in der nach Zugabe eines geeigneten Substrats die Aktivität des Enzyms gemessen wird, z. B. durch den Anstieg der Konzentration mindestens eines Spaltprodukts dieses Substrats über der Zeit.

Solche Vorrichtungen sind in der Praxis vor allem vorteilhaft, wenn AFC als Fluorogen verwendet wird, denn das Emissionsspektrum des abgespalteten Fluorogens ist dann weiter in den langwelligen Bereich verschoben, sodass die Fluoreszenz des abgespaltenen Fluorogens in einem Wellenlängenbereich beobachtet werden kann, in dem andere Lumineszenzen aus der zu untersuchenden Lösung das Messergebnis nicht mehr stören. Gattungsgemäße Vorrichtungen und auch Vorrichtungen der nachfolgend beschriebenen Art sind in dieser Kombination vorteilhaft für die Praxis.

Darüber hinaus lässt sich durch den Einsatz des Bypasses aus zwei Aktivitätsmessungen, nämlich aus der Aktivitätsmessung der Probe nach der Passage durch die Säule und aus der Aktivitätsmessung der Probe nach Passage der Probe durch den Bypass neben der Gesamtaktivität des Enzyms in der Probe auch die Konzentration des Inhibitors bestimmen.

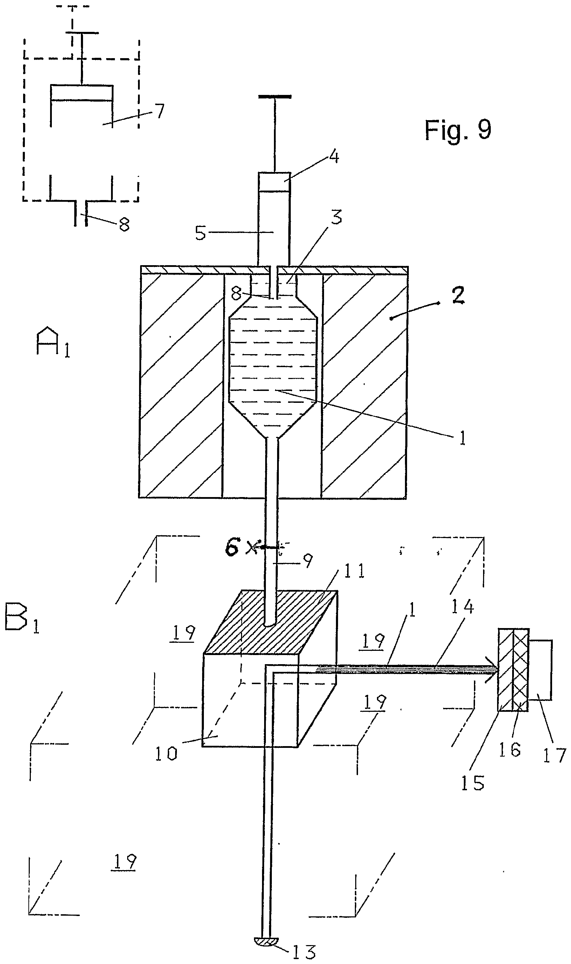

Die Figur 9 zeigt ein erstes einfaches Ausführungsbeispiel der Erfindung.

Dabei ist eine austauschbare Affinitäts-Chromatographiesäule 1 von einem Thermostaten 2 umgeben, der mindestens ein Peltierelement aufweist. Die Säule 1 besteht im wesentlichen aus einem räumlichen Zylinder, der mit poröser Substanz, dem Trägermaterial, ausgefüttert ist, an den eine Inhibitorbindesubstanz gebunden ist. In die obere Öffnung 3 der Säule 1, an deren unterem Ende zweckmäßigerweise ein Sperrventil 6 angebracht ist, ragt die Spitze 8 einer austauschbaren Spritze 4, welche in einem Raum 5 die Messprobe mit dem Enzym-Inhibitor-Komplex enthält.

Das Volumen 5 wird mit ausgedrückter Spritze 4 faktisch zu Null, und der Inhalt entleert sich in die Säule 1, in der sich ein (vorzugsweise fest gepackter) Träger befindet.

Ein Elutionspuffer vom ca. 100-10³fachen Volumen der Messprobe wird danach mittels einer zweiten Spritze 7 ganz oder teilweise in die Säule 1 eingebracht.

Eine erste Vorgehensweise ist folgende: Man belässt die Messprobe mit einem Teil des Elutionspuffers in der Säule 1 bei wohldefinierter Temperatur (z. B. ca. 4° C) für eine bestimmte Inkubationszeit, in der Praxis ca. 10-18 min. Insbesondere 15 min. dürften optimal sein. Danach wird durch weitere Zugabe von Elutionspuffer mit Hilfe der Spritze 7 das freie Enzym eluiert und die Elutionslösung fließt bei offenem Sperrventil 6 nach unten in Modul B gemäß Fig. 9.

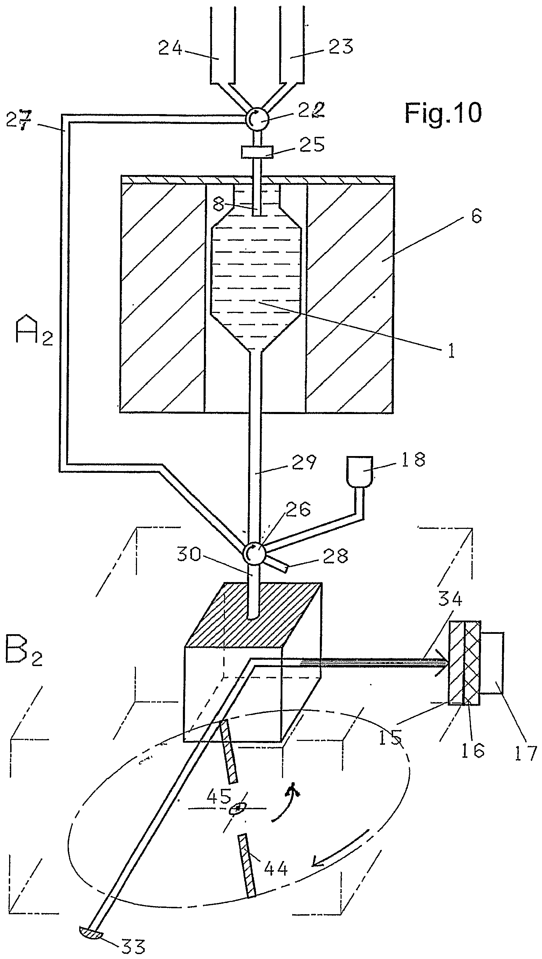

Bei der ersten Methode ist das Ventil 6 zunächst offen, bis der Säulenpuffer in die Säule 1 teilweise eingelaufen ist bzw. bis die Messprobe auf der Säulenlänge verteilt ist. Solange kann auch ein Volumen, das bei der Messung nicht berücksichtigt werden soll, abfließen (z. B. wie in Fig. 10 beschrieben über den Kanal 28). Nach der Inkubationszeit wird zur Elution dieses Ventil 6 geöffnet, dann jedoch wieder geschlossen, damit ein Restvolumen nicht die Messung verschlechtert. Auch dieses Restvolumen kann über den Kanal 28 entsorgt werden.

Eine zweite alternative Maßnahme besteht darin, dass die Messprobe mit Hilfe des zugegebenen Säulenpuffers von oben nach unten durch die Säule wandert mit einer Geschwindigkeit die gewährleistet, dass auf diesem Weg der Inhibitor von allen in der Messprobe enthaltenen Enzym-Inhibitorkomplexen auf die auf der Säule immobilisierte Substanz übergeht, die den Inhibitor stärker bindet als es das Enzym tut. Das ist quasi eine Wanderungsinkubation. Dadurch wird das freie Enzym eluiert und die Elutionslösung fließt nach unten in das Modul B gemäß Fig. 9.

Auch hier wird ein Verwerfungsvolumen anfangs (wie in Fig. 10 mittels Kanal 28 beschrieben) entsorgt; ebenso geschieht dies nach dem Durchlauf des quasi optimalen Messvolumens.

Das Modul A in Fig. 9 stellt also eine Vorrichtung für den Inhibitorentzug dar, die sich im wesentlichen räumlich über der Messbox Modul B befindet, wobei der Durchlauf von der Säule 1 in das Modul B gemäß der Fig. 9 mittels Rohr- oder Schlauchleitung durch einen Deckel 11 hindurch in eine Fluoreszenzküvette 10 zustande kommt. (Der Deckel ist geschwärzt zur Absorption des durch die Messprobe gelangten Laserlichts). Das freie Enzym spaltet gemäß Enzymassay als proteolytisches Enzym aus dem in die Küvette 10 gegebenen Substrat ein Fragment ab, das in freier Form fluoresziert. Aus dem zeitlichen Anstieg dieser Fluoreszenzintensität lässt sich die Enzymaktivität des freigesetzten Enzyms bei definierter Temperatur (welche mit Hilfe von Peltierelementen 19 eingestellt wird) bestimmen. In der unteren Messbox (Modul B) befindet sich eine Laserdiode 13 zur Anregung dieser Fluoreszenz. Die Laserdiode emittiert zweckmäßigerweise Licht der Wellenlänge, die dem Excitationsmaximum des fluoreszierenden Substratfragments entspricht. Das emittierte Fluoreszenzlicht 14 wird senkrecht zur Einstrahlrichtung mit Hilfe einer Fotodiode 17 detektiert. Kantenfilter 15 und Interferenzfilter 16 filtern nahezu alles Streulicht, das vom Anregungslicht stammt, aus und lassen nur Fluoreszenzlicht auf die Fotodiode 17 fallen.

Die Temperierung der Affinitätschromatographiesäule 1 im Modul A erfolgt bei 3-20° C, vorzugsweise bei 4-5° C, da mit der Bindung des Inhibitors an das Affinitäts-Chromatographiesäulen-Material das proteolytische Enzym freigesetzt wird, das sich selbst verdauen kann, d. h. bei höheren Temperaturen greift ein proteolytisches Enzymmolekül das andere an.

Die Messung der Enzymaktivität in Modul B erfolgt temperiert bei 37° C (für humanmedizinische Zwecke), (wozu eine Zusatzeinrichtung für die Konstanthaltung der Temperatur zweckmäßigerweise wiederum mittels Peltierelementen dienlich ist: Thermostat 19).

Im einfachsten Fall ist auch die Küvette 10 als Wegwerfartikel ausgestaltet. (Sie kann vorher gefüllt sein mit Messpuffer und den Ingredientien gemäß Enzymassay). Jedoch kann auch eine Zufuhr dieser Mischung direkt in die Küvette 10 erfolgen, oder u. U. im Kanal 9 mittels zusätzlichem Ventil und Pumpe, je nach Bedarf auch noch mit einem Messpuffer geeigneter Art versetzt. Nach Abschluss der Elution wird die enzymatische Reaktion durch Zugabe von Substrat gestartet. Diese kann z. B., wie in Fig. 10 dargestellt, aus einem Substratbehälter 18 über das Ventil 26 erfolgen, oder wie gemäß Fig. 9, über eine nicht dargestellte Spritze durch den Abschlussdeckel 11 hindurch eingebracht werden.

Die Teile 1, 5, 4, 10 können als (billige) Wegwerfartikel ausgestaltet sein. Der Vorteil der austauschbaren Teile liegt darin, dass keinerlei Bestandteile einer Probe eines Patienten mit einer anderen Probe eines anderen Patienten zusammenkommen können! Man muss sich vor Augen halten, dass die Körperflüssigkeit des Patienten als Messprobe auf die Säule 1 gegeben wird und vor allem nur der Enzym-Inhibitor bei der Elution auf der Säule bleibt; es ist aber nicht klar, ob auch andere Bestandteile auf der Säule noch zurückbleiben. Auf jeden Fall enthält die Messprobe in der Fluoreszenzküvette 10 nicht nur das freie Enzym, sondern auch den größten Teil der anderen Bestandteile der ursprünglichen Körperflüssigkeit. Ein weiterer Vorteil dieses Konzepts besteht darin, dass komplizierte mechanische Teile wie Ventile/ Pumpen entfallen.

Die Verdünnung des Eluats mit Messpuffer kann für ein gutes Messergebnis sehr vorteilhaft, evtl. notwendig sein. Die Kombination von Laserdiode und/oder Fotodiode direkt an der Messküvette führt zu einer hohen Nachweisgrenze. Im einfachsten Fall der Fig. 9 kann man auch so verfahren: Die Elution aus der Säule 1 des Moduls A erfolgt gleich mit dem (im Überschuss beigegebenen) Substrat im Messpuffer, und zwar so, dass jedes Enzym-Molekül ein Substrat-Molekül binden kann (Substratsättigung).

In Fig. 10 ist eine gegenüber der Fig. 9 in der Funktion erweiterte und quasi halbautomatisierbare Vorrichtung dargestellt, jedoch kann auch die Säule 1 wie oben für Fig. 9 mit den zwei Methoden betrieben werden. Daher sind vor und nach der Säule 1 Mehrweg-Ventile 22, 26 vorgesehen. Teile derselben können auch mit einer Anordnung der Fig. 9 kombiniert werden.

In Fig. 10 ist ebenfalls eine Affinitätschromatographiesäule 1, welche von einer temperierbaren Einheit 6 (z. B. Peltierelement) umgeben vorzugsweise auf 4° C eingestellt ist. Im Inneren der Säule ist fest gepacktes Material, an welche eine Substanz gebunden ist, die eine höhere Affinität zu den Inhibitoren aufweist als ein hier interessierender Enzyminhibitorkomplex der Messprobe, z. B. ist an das Sepharosegel als Packungsmaterial Papain als Substanz gebunden, und die Messprobe enthält
z. B. den Enzym-Inhibitorkomplex von Cathepsin B. Nach Einlass der Messprobe aus einem Behälter über einen Tubus 23, welcher über ein Ventil 22 (durch einen Drehpfeil gekennzeichnet) geschieht, wird aus dem Zufluss 24, wobei das Ventil 22 umgestellt wird, ein Säulenpuffer in die Säule 1 gelassen.

Der Tubus 23 kann ein Wegwerfteil sein oder/und als Zufluss aus einem Behälter dienen, der für weitere Messungen benutzt werden kann. Der Kanal 24 kann als eine weitere austauschbare 2. Spritze als Wegwerfteil aus Kunststoff mit einer bestimmten Dosis Volumen, die im allgemeinen das Vielfache vom Volumen der Messprobe beträgt, ausgestattet sein, oder er kann einfach ein Säulenpufferreservoir sein oder zu einem solchen führen, wobei bei der entsprechenden Stellung des Ventils 22 in den Eingang der Säule 1 der Durchfluss für den Säulenpuffer freigegeben wird.

Die Pumpe 25 ist nach dem Ventil 22 angeordnet, um bei Bedarf einen beliebigen Druck (also auch O) zum optimalen Durchlauf durch die Säule zu erzeugen. Das Ventil 22 kann auch so gestellt sein, dass die Messprobe und/oder der Säulenpuffer eine Bypass-Leitung 27 zum unteren Ausgang der Durchflusssäule 1 oder zum Ventil 26 führt. Am Ausgang der Säule 1 ist dieses weitere Ventil 26 vorgesehen (wiederum mit einem Drehpfeil angedeutet), und zwar auch für folgenden Zweck: Wenn die Messprobe durch die Säule 1 läuft, wird ein erster Teil des Volumens verworfen und im Ausgang 28 entsorgt. Sodann wird das Drehventil umgestellt auf Durchflussrichtung 30 zur Messbox B hin, denn dieses weitere Volumen ist dann besser geeignet für eine genaue Messung. Der Rest der Elutionsflüssigkeit wird dann wieder zum Ausgang 28 hin abgeleitet.

Für die Ermittlung des Verwerfungsvolumens wird die Verdünnung der Probe nach dem Durchlauf durch die Säule 1 ermittelt. Eventuell muss man eine einfache Vorrichtung vorsehen zur Bestimmung des Volumens, das verworfen und über den Kanal 28 abgeleitet wird, daraus lässt sich dann das Volumen der eluierten Messprobe mit dem freien Enzym bestimmen als Differenz zu dem über 24 aufgetragenen Elu-tionspuffer.

Nach Durchfluss durch den Kanal 30 in einen (z. B. kubusförmigen oder quaderförmigen) Behälter 10 läuft die Messprobe, deren Enzym von Inhibitoren befreit ist, in diesen Behälter 10, welcher zuvor mit Messpuffer und Ingredientien gemäß Enzym-assay versehen wurde. Zum Start der Enzym-Reaktion wird die optimale Dosis von Substrat zugegeben aus Vorratsbehälter 18.

Der Behälter 10 kann ebenfalls für einfachste Bedürfnisse als Wegwerfartikel ausgestaltet sein, indem der Kubus 10 z. B. aus Kunststoff besteht.

Er kann eine Fluoreszenzküvette sein, wobei im Modul B eine Laserdiode 33 vorgesehen ist, die eine Wellenlänge λ = 400 nm aufweist.

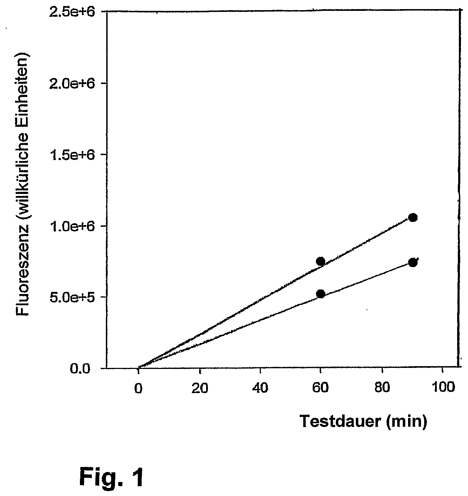

Diese Laserstrahlung aus 33 ist etwa senkrecht zur Fallrichtung, d. h. etwa 90° zur Einflussrichtung vom Kanal 29 oder 30. Senkrecht dazu (vom Betrachter aus nach rechts im Winkel von 90°) ist als Reaktion des Spaltprodukts eine Fluoreszenzstrahlung 34 dargestellt, die auf einen Kantenfilter 15, parallel dazu einen Interferenzfilter 16, und weiter parallel dazu auf eine Fotodiode 17 trifft, die an ihrem Ausgang die Intensität der Fluoreszenzstrahlung zu messen gestattet (wie in Fig. 1). Die Strahlungen 34, 35 liegen also in einer Ebene, die vorzugsweise senkrecht zur Fallrichtung ist.

Unterhalb der Fluoreszenzküvette 10 ist ein Magnetrührgerät 44 um die Achse 45 rotierend angedeutet, das die Mischung möglichst homogen machen soll. Fluoreszenz beginnt sofort, nachdem die Partikel der Messprobe auf Substratteile treffen, die Intensität ist proportional der Konzentration des Spaltprodukts und dieses wiederum ist ein proportionales Maß für die Enzymaktivität.

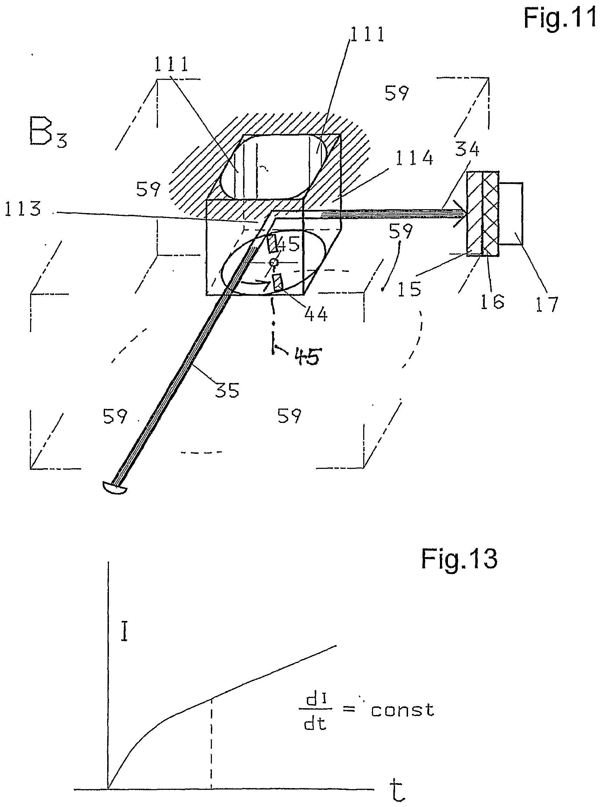

Die Messkurve für die emittierte Fluoreszenzintensität in Fig. 13 geht nach einer Anfangsphase in eine Gerade über, und sobald der Kurvenverlauf geradlinig ist, wird deren Anstieg festgestellt und man hat das gewünschte Ergebnis dl/dt. Die Fig. 13 ist für alle anderen Figuren repräsentativ.

Im einfachsten Fall ist das Modul A eine Wegwerf-Chromatographiesäule, in welche oben eine erste Einmalspritze mit der Messprobe eingeführt wird, danach eine 2. Spritze mit dem Säulenpuffer, der Ausgang 9 kann unmittelbar über das Ventil 6 mit der Messbox 10 verbunden werden, wobei diese dann auch eine (wie beschrieben) aufbereitete, billige Wegwerfpackung ist. Das Modul B, auf jeden Fall die Messbox 10, sollte temperiert werden, wozu um die Messbox 10 herum ein weiteres Peltier-element 16 in unmittelbarer Umgebung vorgesehen ist. (Das muss für humanmedizinische Anwendung zweckmäßigerweise auf 37° C eingestellt sein). Alternativ kann also ein Substratbehälter 18 vorgesehen sein, der einen direkten Kanal zur Box 10 hin aufweist. Vorzugsweise erfolgt die Zufuhr zur Box 10 jedoch aus dem Substratbehälter indirekt über den Kanal 9 bzw. 29 bzw. 30.

Im einfachsten Fall ist Modul B eine mit Messpuffer und weiteren Ingredientien, wie z. B. ein nicht ionisches Tensid und Dithiothreitol oder Cystein, aufbereitete billige Wegwerfpackung.

Das Ventil ist dann unerlässlich, wenn man die Messprobe im Durchflussverfahren aus der Säule eluiert, denn dann wird man einen ersten Teil des Eluats verwerfen. Es ist aber auch dann unerlässlich, wenn man die Probe auf der Säule eine Zeit lang inkubiert; denn dazu muss man nach dem Auftragen der Probe auf die Säule danach einen gewissen Teil des Säulenpuffers in die Säule lassen und unten auslassen; dadurch sickert die Messprobe in die Säule ein und die Enzym-Inhibitorkomplexe der Messprobe finden so möglichst alle Kontakt mit der auf der Säule immobilisierten Substanz, die den Inhibitor besser bindet,

Während in der Anordnung gemäß Fig. 9 die Laserstrahlung 14 und das emittierte und detektierte Fluoreszenzlicht parallel oder in der Zeichnungsebene bzw. Schnittebene von Modul A verlaufen, liegen diese Strahlungen 34, 35 in Fig. 10 in einer Ebene senkrecht zur Schnittebene von Modul A, d. h. im allgemeinen senkrecht zur Fallrichtung oder Durchflussrichtung in Teil 1.

Dadurch ist unterhalb der Messküvette 10 bzw. des Moduls B ein Mischer zur Homogenisierung für den Inhalt der Küvette 10 räumlich geschickt und für einfache Handhabung vorteilhaft angeordnet, z. B. als magnetisches Rührwerk 44, 45.

In diesen Figurenbeschreibungen sind identische Ziffern für gleich wirkende oder identische Teile verwendet. Außerdem wird Modul A als ebenes Schnittbild dargestellt, während Modul B in räumlicher Darstellung gezeigt ist.

Fig. 11 stellt eine weitere selbständige Variante der Erfindung dar, bei der Modul A entfällt und die Messfunktion komplett in Modul B verlagert ist. Die Messküvette ist quasi als Messtopf 100 ausgebildet, der einen (wie schon beschrieben) geschwärzten Abschluss 111 aufweist, welcher vorzugsweise Öffnungen für (insbesondere automatisierbare) Zufuhr der notwendigen Substanzen aufweist. Hierbei wird das Affinitätschromatographieadsorbens (z. B. Papain + Trägermaterial) als Gel bereits mit Messpuffer zusammen mit den übrigen Ingredientien des Enzymassays in den Messtopf gegeben, der z. B. wieder als Fluoreszenzküvette ausgestattet ist. Nach Zugabe der biologischen Messprobe wird bei der für die Inkubation optimalen Temperatur von z. B. 5° C, welche mittels Peltierelementen gehalten wird, die optimale Zeit lang inkubiert und dabei sozusagen in situ das vom Inhibitor freie Enzym in der Fluoreszenzküvette freigesetzt. Danach wird dieses Gemisch auf die für den Enzymassay notwendige Temperatur, (die für humanmedizinische Zwecke 37° C beträgt), gebracht und nach Erreichen dieser Temperatur das fluorogene Substrat zugespritzt, sodass die Fluoreszenzintensität und damit die Enzymaktivität des freien proteolytischen Enzyms gemessen werden kann (s. Fig. 13).

Das Verfahren, das gemäß Fig. 11 arbeitet, sieht also an ortsfester Stelle (im Messtopf 100 Modul B) vor, dass zuerst die Inkubation bei niedrigerer Temperatur geschieht, sodann die Temperatur hochgefahren wird (z. B. 37 ° C), und dann das Substrat zugegeben wird.

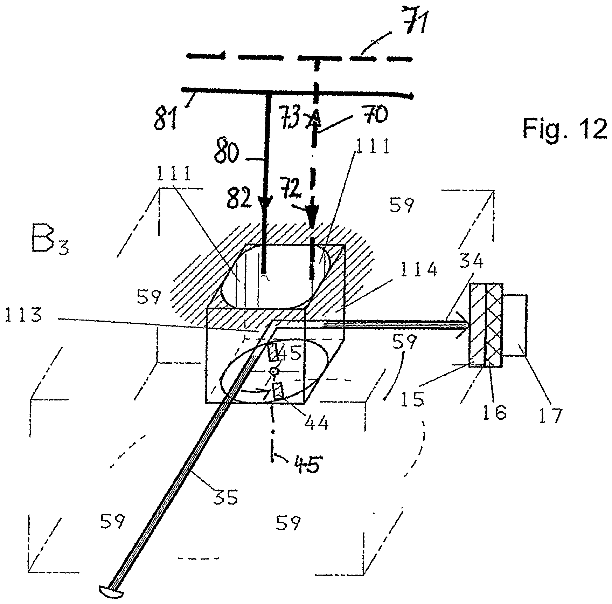

Fig. 12 stellt eine noch weitere selbstständige Variante der Erfindung dar, bei der Modul A entfällt und die Messfunktion komplett in Modul B verlagert ist, in welcher sich die Messprobe befindet. Die Messküvette ist wie in Fig. 11 quasi als Messtopf 100 ausgebildet. Hierbei wird das Affinitätschromatographieadsorbens (z. B. Papain + festes Trägermaterial) auf einem festen Einsatz 71 in Richtung des Pfeils 72/70 in die Messprobe im Messtopf eingetaucht, der z. B. wieder als Fluoreszenzküvette ausgestattet ist. Nach der für die Inkubation (Deinhibition) optimalen Zeit wird der feste Einsatz in Richtung Pfeil 73 entfernt. Danach wird die Messprobe auf die für den Enzymassay notwendige Temperatur, (die für humanmedizinische Zwecke 37 °C beträgt), gebracht und nach Erreichen dieser Temperatur das fluorogene Substrat zugegeben, zugespritzt, sodass die Zunahme der Fluoreszenzintensität und damit die Enzymaktivität des freien proteolytischen Enzyms gemessen werden kann (s. Fig. 13).

Das Verfahren, das gemäß Fig. 12 arbeitet, sieht also an ortsfester Stelle (im Messtopf 100 Modul B) vor, dass zuerst die Inkubation bei niedrigerer Temperatur geschieht, sodann die Temperatur hochgefahren wird (z. B. 37 °C), und dann das Substrat zugegeben wird.

## Patentansprüche

1. Verfahren zur Bestimmung der Aktivität wenigstens einer Cystein-Protease in einer flüssigen unter Blutplasma und Blutserum ausgewählten Messprobe, welche neben der wenigstens einen Cystein-Protease, deren Aktivität zu bestimmen ist, gegebenenfalls wenigstens einen zu der Protease korrespondierenden Proteaseinhibitor enthält, mit den Stufen, in denen man:
der Cystein-Protease in der Messprobe den Proteaseinhibitor entzieht, indem man die Messprobe mit einem festen oder gelartigen Trägermaterial in Kontakt bringt, an welchem eine Inhibitorbindesubstanz kovalent oder adsorptiv gebunden ist, die eine höhere Affinität / Bindungsstärke zu dem Proteaseinhibitor besitzt als die Cystein-Protease selbst,
das Trägermaterial mit daran gebundenem Proteaseinhibitor von der Messprobe separiert, der Messprobe ein Substrat für die wenigstens eine Cystein-Protease, deren Aktivität zu bestimmen ist, zugibt und die proteolytische Umsetzung des Substrates durch die Cystein-Protease erfasst,
wobei das Substrat für die wenigstens eine Cystein-Protease eine Di- oder Oligopeptidsequenz umfasst, an deren C-Terminus das Fluorogen 7-Amino-4-trifluormethylcumarin (AFC) gebunden ist.

2. Verfahren nach Anspruch 1, **dadurch gekennzeichnet, dass** die wenigstens eine Cystein-Protease ausgewählt ist unter einem der Cathepsine B, H, K, L und/oder S, wobei die Cystein-Protease besonders bevorzugt Cathepsin B ist.

3. Verfahren nach einem der vorangegangenen Ansprüche, **dadurch gekennzeichnet, dass** die flüssige Messprobe Blutserum ist.

4. Verfahren nach Anspruch einem der vorangegangenen Ansprüche, **dadurch gekennzeichnet, dass** das Substrat, welches die Di- oder Oligopeptidsequenz und das Fluorogen umfasst, ein Maximum der Fluoreszenzemissionswellenlänge besitzt, das sich von dem Maximum der Fluoreszenzemissionswellenlänge des durch die Cystein-Protease bei der proteolytischen Reaktion abgespaltenen Fluorogens um wenigstens 20 nm, vorzugsweise wenigstens 40 nm oder wenigstens 60 nm oder wenigstens 80 nm, besonders bevorzugt wenigstens 100 nm unterscheidet.

5. Verfahren nach einem der vorangegangenen Ansprüche, **dadurch gekennzeichnet, dass** der N-Terminus der Di- oder Oligopeptidsequenz eine Schutzgruppe aufweist.

6. Verfahren nach einem der vorherigen Ansprüche, **dadurch gekennzeichnet, dass** man den zeitlichen Verlauf der Konzentrationsveränderung des Produktes der proteolytischen Umsetzung des Substrats, d. h. des abgespaltenen Fluorogens, durch die Cystein-Protease, vorzugsweise fluorometrisch, erfasst.

7. Verfahren nach Anspruch 6, **dadurch gekennzeichnet, dass** die Veränderung der Konzentration im linearen Bereich bei 37 °C gemessen wird.

8. Verfahren nach einem der vorherigen Ansprüche, **dadurch gekennzeichnet, dass** man einem Aliquot der Messprobe das Substrat der Cystein-Protease zugibt und die Enzymaktivität ohne vorherige Deinhibierung misst und das andere Aliquot der Messprobe vor der eigentlichen Enzymaktivitätsmessung mit dem Trägermaterial in Kontakt bringt, an dem die Inhibitorbindesubstanz haftet, wodurch der Anteil der Aktivität des inhibierten Enzyms sich aus der Differenz der beiden Messwerte ergibt.

9. Verfahren nach Anspruch 8, **dadurch gekennzeichnet, dass** das feste Trägermaterial Nylon oder Nitrozellulose umfasst oder daraus hergestellt ist und vorzugsweise als Folie oder Membran ausgebildet ist.

10. Verfahren nach einem der vorhergehenden Ansprüche, **dadurch gekennzeichnet, dass** die Inhibitorbindesubstanz Papain ist.

11. Verfahren nach einem der vorhergehenden Ansprüche, **dadurch gekennzeichnet, dass** die Messprobe bei gelartigem Trägermaterial für eine Zeitspanne von höchstens 5 Minuten, vorzugsweise höchstens 2 Minuten bei einer Temperatur im Bereich von 15 bis 40 °C, vorzugsweise 20 bis 35 °C, besonders bevorzugt 20 bis 30 °C oder für eine Zeitspanne von 10 -48 Minuten bei 4 °C mit dem Trägermaterial in Kontakt gebracht wird, bevor das Trägermaterial von der Messprobe separiert wird

12. Verfahren nach einem der vorhergehenden Ansprüche, insbesondere nach Anspruch 11, **dadurch gekennzeichnet, dass** die Zeitwerte bei festem Trägermaterial größer als die in Anspruch 11 genannten sind, insbesondere jedoch dass die Messprobe bei festem Trägermaterial für eine Zeitspanne von ca. 10 Minuten, vorzugsweise ca. 4 Minuten bei einer Temperatur im Bereich von 15 bis 40 °C, vorzugsweise 20 bis 35 °C, besonders bevorzugt 20 bis 30 °C mit dem Trägermaterial in Kontakt gebracht wird, bevor das Trägermaterial von der Messprobe separiert wird.

## Claims

1. Method for measuring the activity of at least one cysteine protease in a fluid sample selected of blood plasma or blood serum, which contains besides the at least one cysteine protease, the activity of which is to be measured, at least one protease inhibitor corresponding to the said protease, comprising the following steps in which:
from the cysteine protease in the sample the protease inhibitor is withdrawn, wherein the sample is brought into contact with a rigid or gel-like carrier, to which an inhibitor binding substance is bound covalently or in an adsorptive manner that has a higher affinity or binding strength to the protease inhibitor than the cysteine protease,
the carrier with the protease inhibitor bound to it is separated from the sample, a substrate for the at least one cysteine protease, the activity of which is to be measured, is added to the sample and the proteolytic reaction of the substrate with the cysteine protease is recorded,
wherein the substrate for the at least one cysteine protease includes a di- or oligopeptide sequence, to the C-terminus of which the fluorogen 7-amino-4-trifluoromethylcoumarin (AFC) is bound.

2. Method according to claim 1 **characterized in that** the at least one cysteine protease is selected from one of the cathepsins B, H, K, L and/or S, whereby the cysteine protease cathepsin B is especially preferred.

3. Method according to one of the preceding claims **characterized in that** the fluid sample is blood serum.

4. Method according to one of the preceding claims, **characterized in that** the substrate, which includes the di- or oligopeptide sequence and the fluorogen, has a maximum of its fluorescence emission wavelength, which differs from the maximum of the fluorescence emission wavelength of the fluorogen being cleaved by the protease in the proteolytic reaction in at least 20 nm, preferably in at least 40 nm or in at least 60 nm or in at least 80 nm, especially preferred in at least 100 nm.

5. Method according to one of the preceding claims **characterized in that** the N-terminus of the di- or oligopeptide sequence has a protecting group.

6. Method according to one of the preceding claims **characterized in that** the time course of the concentration change of the product of the proteolytic reaction of the substrate with the cysteine protease, i.e. of the cleaved fluorogen, is preferably recorded fluorometrically.

7. Method according to claim 6 **characterized in that** the concentration change is measured in the linear range at 37 °C.

8. Method according to one of the preceding claims **characterized in that** the substrate of the cysteine protease is added to an aliquot of the sample and the enzyme activity is measured without preceding deinhibition, and the other aliquot of the sample is prior to the enzyme activity measurement brought into contact with the carrier, to which the inhibitor binding substance is bound, and the portion of the activity of the inhibited enzyme results from the difference of the two measured values.

9. Method according to claim 8 **characterized in that** the rigid carrier includes nylon or nitrocellulose or is made thereof and is preferably shaped as a foil or a membrane.

10. Method according to one of the preceding claims **characterized in that** the inhibitor binding substance is papain.

11. Method according to one of the preceding claims **characterized in that** the sample in case of a gel-like carrier is brought into contact with the carrier for not more than five minutes, preferably not more than two minutes in a temperature range of 15 to 40 °C, preferably between 20 - 35 °C, especially preferably between 20 to 30 °C, or for a time of 10 - 48 minutes at 4 °C, and afterwards the carrier is separated from the sample.

12. Method according to one of the preceding claims, especially according to claim 11, **characterized in that** the time range in case of a rigid carrier is greater than specified in claim 11, and especially that the sample in case of a rigid carrier is brought into contact with the carrier for a time range of ca. 10 minutes, preferably of ca. 4 minutes at a temperature range between 15 to 40 °C, preferably between 20 to 35 °C, especially preferably between 20 to 30 °C, and afterwards the carrier is separated from the sample.

## Revendications

1. Procédé de détermination de l'activité d'au moins une cystéine-protéase dans un échantillon de mesure fluide sélectionné parmi du plasma sanguin et du sérum sanguin, lequel contient, en plus de la cystéine-protéase, au moins au nombre de une, dont l'activité doit être déterminée, éventuellement au moins un inhibiteur de protéase correspondant à la protéase, comprenant les étapes au cours desquelles :
on retire de la cystéine-protéase dans l'échantillon de mesure l'inhibiteur de protéase, en ce que l'on met en contact l'échantillon de mesure avec un matériau de support solide ou de type gel auquel est liée de façon covalente ou par adsorption une substance de liaison de l'inhibiteur qui possède une plus grande affinité / force de liaison à l'inhibiteur de protéase que la cystéine-protéase elle-même,
on sépare de l'échantillon de mesure le matériau de support avec l'inhibiteur de protéase qui y est lié,
on ajoute à l'échantillon de mesure un substrat pour la cystéine-protéase, au moins au nombre de une, dont l'activité doit être déterminée, et on enregistre la conversion protéolytique du substrat par la cystéine-protéase, où le substrat pour la cystéine-protéase, au moins au nombre de une, comprend une séquence di- ou oligo-peptidique, à l'extrémité C-terminale de laquelle est lié le fluorogène 7-amino-4-trifluorométhylcoumarine (AFC).

2. Procédé selon la revendication 1, **caractérisé en ce que** la cystéine-protéase, au moins au nombre de une, est sélectionnée parmi une des cathepsines B, H, L, L et/ou S, où la cystéine-protéase est de façon particulièrement préférée la cathepsine B.

3. Procédé selon l'une des revendications précédentes, **caractérisé en ce que** l'échantillon de mesure fluide est du sérum sanguin.

4. Procédé selon l'une des revendications précédentes, **caractérisé en ce que** le substrat qui comprend la séquence di- ou oligo-peptidique et le fluorogène possède un maximum de longueur d'onde d'émission de fluorescence qui se distingue du maximum de longueur d'onde d'émission de fluorescence du fluorogène séparé par la cystéine-protéase lors de la réaction protéolytique d'au moins 20 nm, de préférence d'au moins 40 nm ou d'au moins 60 nm ou d'au moins 80 nm, et de façon particulièrement préférée d'au moins 100 nm.

5. Procédé selon l'une des revendications précédentes, **caractérisé en ce que** l'extrémité N-terminale de la séquence di- ou oligo-peptidique présente un groupe protecteur.

6. Procédé selon l'une des revendications précédentes, **caractérisé en ce que** l'on enregistre l'évolution temporelle de la modification de concentration du produit de la conversion protéolytique du substrat, à savoir du fluorogène séparé, par la cystéine-protéase, de préférence par voie fluorométrique.

7. Procédé selon la revendication 6, **caractérisé en ce que** la modification de la concentration est mesurée dans la plage linéaire à 37 °C.

8. Procédé selon l'une des revendications précédentes, **caractérisé en ce que** l'on ajoute le substrat de la cystéine-protéase à une fraction aliquote de l'échantillon de mesure et que l'on mesure l'activité enzymatique sans désinhibition préalable, et que l'on met en contact l'autre fraction aliquote de l'échantillon de mesure, avant la mesure de l'activité enzymatique à proprement parler, avec le matériau de support auquel adhère la substance de liaison de l'inhibiteur, moyennant quoi la fraction de l'activité de l'enzyme inhibée découle de la différence des deux valeurs de mesure.

9. Procédé selon la revendication 8, **caractérisé en ce que** le matériau de support solide comprend ou est fabriqué à partir de nylon ou de nitrocellulose, et est de préférence configuré en tant que film ou membrane.

10. Procédé selon l'une des revendications précédentes, **caractérisé en ce que** la substance de liaison de l'inhibiteur est la papaïne.

11. Procédé selon l'une des revendications précédentes, **caractérisé en ce que** l'échantillon de mesure est mis en contact, dans le cas d'un matériau de support de type gel, pendant une durée d'au plus 5 minutes, de préférence d'au plus 2 minutes, à une température dans la plage de 15 à 40 °C, de préférence de 20 à 35 °C, de façon particulièrement préférée de 20 à 30 °C, ou est mis en contact pendant une durée de 10 à 48 minutes à 4 °C avec le matériau de support avant que le matériau de support soit séparé de l'échantillon de mesure.

12. Procédé selon l'une des revendications précédentes, en particulier selon la revendication 11, **caractérisé en ce que** les durées, dans le cas d'un matériau de support solide, sont supérieures à celles nommées dans la revendication 11, en particulier cependant **en ce que** l'échantillon de mesure, dans le cas d'un matériau de support solide, est mis en contact pendant une durée d'environ 10 minutes, de préférence d'environ 4 minutes, à une température dans la plage de 15 à 40 °C, de préférence de 20 à 35 °C, de façon particulièrement préférée de 20 à 30 °C, avec le matériau de support avant que le matériau de support soit séparé de l'échantillon de mesure.
